# EUROPÄISCHE PATENTSCHRIFT

(11) **EP 1 879 966 B1**
(45) Veröffentlichungstag und Bekanntmachung des Hinweises auf die Patenterteilung: **04.03.2009**
(21) Anmeldenummer: 06754980.8
(22) Anmeldetag: 03.05.2006
(51) Int. Cl.: C09D 5/00, C08G 73/00, C08F 8/00

(54) **BIOZIDE BESCHICHTUNGEN**
BIOCIDAL COATINGS
REVETEMENTS BIOCIDES

(30) Priorität: 04.05.2005 DE 102005021363
(43) Veröffentlichungstag der Anmeldung: 23.01.2008
(73) Patentinhaber: BASF SE, 67056 Ludwigshafen (DE)
(72) Erfinder: CHAMP, Simon, 67063 Ludwigshafen (DE); SEYFFER, Hermann, 69123 Heidelberg (DE)
(86) Internationale Anmeldenummer: PCT/EP2006/062014
(87) Internationale Veröffentlichungsnummer: WO 2006/117382

(56) Entgegenhaltungen:
- WO-A-01/53359
- WO-A-99/12424
- WO-A-20/04032628
- RU-C1- 2 181 808

## Beschreibung

Die Erfindung betrifft die Verwendung eines kationischen Polymeren als biozider Wirkstoff, dadurch gekennzeichnet, dass keine sonstigen bioziden Wirkstoffe, insbesondere keine quarternären Ammoniumsalze, mitverwendet werden, es sich bei dem Polymer um ein Polyethylenimin handelt, welches zu mindestens 95 Gew. % aus Einheiten der Formel

-CH2-CH2-N I

besteht, worin N für ein primäres, sekundäres, tertiäres oder quarternäres Stickstoffatom steht, oder um ein ein Polyvinylamin handelt, welches aus mindestens 95 Gew.-% ethylenisch ungesättigten, radikalisch polymerisierbaren Verbindungen mit einer primären, sekundären, tertiären oder quarternären Aminogruppe in polymerisierter Form besteht und das Polymer 0,1 bis 22 Milliequivalent kationische Gruppen pro Gramm Polymer enthält.

Gegenstand der Erfindung sind auch mit dem bioziden Wirkstoff ausgerüstete Substrate, welche durch Beschichtung, Imprägnierung oder sonstige Behandlung der gewünschten Substrate mit dem Polymer, bzw. der Lösung oder Dispersion des Polymeren, erhältlich sind.

Biozide Wirkstoffe töten Mikroorganismen, wie Bakterien, Pilze, Hefen, Algen oder Viren ab oder verhindern zumindest deren Reproduktion und/oder Wachstum.

Bei unterschiedlichsten Substraten besteht der Wunsch oder auch oft die Notwendigkeit einer bioziden Ausrüstung. Es handelt sich dabei z.B. um Substrate für medizinische Anwendungen, Anwendungen im Sanitär- oder Hygienebereich, im Lebensmittelbereich, insbesondere bei Lebensmittelverpackungen, oder Substrate für vielfältige industrielle Anwendungen, insbesondere Filter, z.B. für Klimaanlagen.

Aus WO 2004/087226 sind derartige, mit bioziden Wirkstoffen ausgerüstete Substrate bekannt. Als biozider Wirkstoff wird eine Mischung von kationischen Polymeren und speziellen quaternären Ammoniumsalzen eingesetzt.

Aus US 6261581, bzw. DE-A-19608555, ist die Verwendung von Polyvinylamin- und Polyethylenimincopolymeren als biozider Wirkstoff bekannt. Polyvinylamin- und Polyethyleniminhomopolymere fallen unter die in US 6261581 angegebene Formel, in der Beschreibung werden jedoch in konkreter Form nur Copolymere offenbart.

In der WO 2004/032628 wird ein Verfahren zum Abtöten von Mikrooranismen beschrieben. Als Wirkstoff wird ein Polymer der Styrolsulfonsäure eingesetzt. Hierbei handelt es sich entweder um ein Homopolymer mit 100 mol% Styrolsulfonsäure oder um ein Copolymer aus mindestens 40 mol% Styrolsulfonsäure und 0 bis 40 mol% N-Vinyllactam und/oder N-Vinylamin und 0 bis 30 mol% weiterer radikalisch polymerisierbarer Monomerer. In der RU 2181808 wird eine Zusammensetzung zur Herstellung von biozidem Papier beschrieben. Als Wirkstoff wird ein aus zwei Komponenten bestehender Wirkstoffkomplex eingesetzt, wobei die beiden Wirkkomponenten Polyhexamethylen-guanidin und Polyethylenimin in einem Verhältnis von 100:1 bis 1:100 eingesetzt werden. In der WO 01/53359 werden vernetzte, in Wasser unlösliche Gele beschrieben. Die Gele werden hergestellt aus Polyethylenimin und nicht hydrolisiertem, cyclischem N-Vinyllactampolymer. Die Gele können u. a. eingesetzt werden als adhäsive Basis für biozide Wirkstoffkomponenten. In der WO 99/12424 wird die Verwendung bestimmter wasserlöslicher Polymerisate als Biozide beschrieben. Die Polymerisate werden hergestellt durch Umsetzung von Polyetheylenimin mit Halogenameisensäureestern zu Polyethylencarbamaten.

Aufgabe der vorliegenden Erfindung waren kostengünstigere biozide Wirkstoffe und ein einfacheres Verfahren zur bioziden Ausrüstung von Substraten.

Demgemäß wurde die eingangs definierte Verwendung gefunden.

### Zu den Polyethyleniminen

Bevorzugt bestehen die Polyethylenimine zu mindestens 99 Gew.-% , besonders bevorzugt zu mindestens 100 Gew.% aus den obigen Einheiten CH2-CH2-N.

Das Stickstoffatom N kann ein primäres, sekundäres oder tertiäres Stickstoffatom sein. Primäre Stickstoffatome können insbesondere als NH2 Gruppe am Kettenende stehen, sekundäre N Atome sind als -NH- Bestandteil von Polymerketten und tertiäre N Atome sind Vernetzungspunkte, an die 3 CH2-CH2 Gruppen gebunden sind.

Ein Teil der N Atome trägt eine positive Ladung, ist also quaternisiert. Eine Quaternisierung der N Atome kann durch Einstellung des ph wertes erreicht werden, im sauren wird ein entsprechender Teil der N Atome protoniert.

Eine Quaternisierung der Polyethylenimine kann beispielsweise auch mit Alkylhalogeniden wie Methylchlorid, Ethylchlorid, Hexylchlorid, Benzylchlorid oder Laurylchlorid sowie mit beispielsweise Dimethylsulfat vorgenommen werden.

Die Homopolymerisate werden beispielsweise durch Polymerisieren von Ethylenimin in wässriger Lösung in Gegenwart von Säuren, Lewis-Säuren oder Alkylierungsmitteln wie Methylchlorid, Ethylchlorid, Propylchlorid, Ethylenchlorid, Chloroform oder Tetrachlorethylen hergestellt. Die so erhältlichen Polyethylenimine haben eine breite Molmassenverteilung und mittlere Molmassen Mw, von beispielsweise 120 bis 2-106. vorzugsweise 430 bis 1-106. Die mittleren Molmassen Mw der in Betracht kommenden Polyethylenimine können bis zu 2 Mio. betragen und liegen vorzugsweise in dem Bereich von 1 000 bis 50 000.

Vorzugsweise enthält das Polyethylenimin 0,1 bis 22 Milliequivalent, besonders bevorzugt 4 bis 10 Milliequivalent kationische Gruppen pro Gramm Polyethylenimin.

### Zu den Polyvinylaminen

Bei dem Polyvinylamin handelt es sich um ein Polymer, welches aus mindestens 95 Gew.-%, vorzugsweise zu mindestens 99 Gew.-% und ganz besonders bevorzugt zu 100 Gew.-% aus ethylenisch ungesättigten, radikalisch polymerisierbaren Verbindungen mit einer primären, sekundären, tertiären oder quaternären Aminogruppe aufgebaut ist, d.h. durch Polymerisation aus diesen Monomeren erhältlich ist.

Insbesondere handelt es sich bei derartigen Monomeren um Vinylcarbonsäureamide, welche insbesondere sekundäre und tertiäre Aminigruppen in Form von subtstituierten Amidgruppen enthalten und die aus diesen Vinylcarbonsäureamiden durch Hydrolyse erhältlichen Monomeren mit primären Aminogruppen.

Polymere, wie sie im Sinne der vorliegenden Erfindung eingesetzt werden, sind bekannt, vgl. US 4,421,602. US 5,334,287, EP-A 216 387, US 5,981,689, WO 00/63295, US 6,121,409 und US 6,132,558. Sie werden im allgemeinen durch Hydrolyse von offenkettigen N-Vinylcarbonsäure-amideinheiten enthaltenden Polymeren hergestellt. Diese Polymeren sind z.B. erhältlich durch Polymerisieren von N-Vinylformamid, N-Vinyl-N-methylformamid, N-Vinyl-acetamid, N-Vinyl-N-methylacetamid, N-Vinyl-N-ethylacetamid und N-Vinylpropionamid. Die genannten Monomeren können entweder allein oder zusammen mit anderen Monomeren polymerisiert werden. Bevorzugt ist N-Vinylformamid.

Die Polymerisation der Monomeren wird üblicherweise in Gegenwart von Radikale bildenden Polymerisationsinitiatoren durchgeführt. Man kann die Homo- und Copolymerisate nach allen bekannten Verfahren erhalten, beispielsweise erhält man sie durch Lösungspolymerisation in Wasser, Alkoholen, Ethern oder Dimethylformamid oder in Gemischen aus verschiedenen Lösungsmitteln, durch Fällungspolymerisation, umgekehrte Suspensionspoylmerisation (Polymerisieren einer Emulsion einer monomerhaltigen wässrigen Phase in einer Ölphase) und Polymerisieren einer Wasser-in-Wasser-Emulsion, beispielsweise bei der man eine wässrige Monomerlösung in einer wässrigen Phase löst oder emulgiert und unter Bildung einer wässrigen Dispersion eines wasserlöslichen Polymeren polymerisiert, wie beispielsweise in WO 00/27893 beschrieben. Im Anschluss an die Polymerisation werden die Homo- und Copopolymerisate, die einpolymerisierte N-Vinylcarbonsäureamideinheiten enthalten, wie unten beschrieben partiell oder vollständig hydrolysiert.

Vorzugsweise geht man von Homopolymerisaten des N-Vinylformamids oder von Copolymerisaten von Vinylcarbonsäureamide, insbesondere N-Vinylformamid, mit anderen Vinyöcarbonsäureamiden aus, durch anschließende Hydrolyse der Homo- oder der Copolymerisate bilden sich Einheiten mit primären Aminogruppen aus, wobei der Hydrolysegrad z.B. 1 bis 100 mol-%, vorzugsweise 25 bis 100 mol-%, besonders bevorzugt 50 bis 100 mol-% und insbesondere bevorzugt 70 bis 100 mol-% beträgt. Der Hydrolysegrad entspricht dem Gehalt der Polymeren an primären Vinylamingruppen in mol-%. Die Hydrolyse der oben beschriebenen Polymerisate erfolgt nach bekannten Verfahren durch Einwirkung von Säuren (z.B. Mineralsäuren wie Schwefelsäure, Salzsäure oder Phosphorsäure, Carbonsäuren wie Ameisensäure oder Essigsäure, bzw. Sulfonsäuren oder Phsophonsäuren), Basen oder Enzymen, wie beispielsweise in DE-A 31 28 478 und US 6,132,558 beschrieben. Bei Verwendung von Säuren als Hydrolysemittel liegen die Vinylamineinheiten der Polymerisate als Ammoniumsalz vor, während bei der Hydrolyse mit Basen die freie Aminogruppen entstehen.

Die mittleren Molmassen Mw der Vinylamineinheiten enthaltenden Polymerisate betragen z.B. 500 bis 10 Millionen, vorzugsweise 750 bis 5 Millionen und besonders bevorzugt 1 000 bis 2 Millionen g/mol (bestimmt durch Lichtstreuung). Dieser Molmassenbereich entspricht beispielsweise K-Werten von 30 bis 150, vorzugsweise 60 bis 100 (bestimmt nach H. Fikentscher in 5 %iger wässriger Kochsalzlösung bei 25 °C, einen pH-Wert von 7 und einer Polymerkonzentration von 0,5 Gew.-%). Besonders bevorzugt werden Vinylamineinheiten enthaltende Polymere eingesetzt, die K-Werte von 85 bis 95 haben.

Vorzugsweise enthält das Polyvinylamin 0,1 bis 22 Milliequivalent (meq), besonders bevorzugt 10 bis 20 meq kationische Gruppen pro Gramm Polyvinylamin.

Die Vinylamineinheiten enthaltenden Polymeren werden vorzugsweise in salzfreier Form eingesetzt. Salzfreie wässrige Lösungen von Vinylamineinheiten enthaltenden Polymerisaten können beispielsweise aus den oben beschriebenen salzhaltigen Polymerlösungen mit Hilfe einer Ultrafiltration an geeigneten Membranen bei Trenngrenzen von beispielsweise 1 000 bis 500 000 Dalton, vorzugsweise 10 000 bis 300 000 Dalton hergestellt werden.

Bevorzugt in Betracht kommende Polyvinylamine sind Homopolymere des N-Vinylformamids mit einem Hydrolysegrad von 1 bis 100 mol %, bevorzugt 25 bis 100 mol-%, sowie zu 1 bis 100 mol-%.

Typische Vertreter diese Homopolymerisate des N-Vinylformamids sind unter den Handelsnamen Catiofast® VFH, Catiofast® VSH und Catiofast® VMP der BASF Aktiengesellschaft bekannt.

Das Polyethylenimin und Polyvinylamin wird vorzugsweise in Form einer wässrigen Dispersion oder Lösung verwendet.

Biozid ausgerüstete Substrate sind dadurch erhältlich, dass die gewünschten Substrate mit dem Polethylenimin oder dem Polyvinylamin, bzw. dessen Lösung oder Dispersion, beschichtet, imprägniert oder sonst wie behandelt werden. Die Behandlung mit der Lösung oder Dispersion kann bei Raumtemperatur erfolgen, nach der Trocknung ist das Substrat entsprechend ausgerüstet.

Die Menge an Polymer beträgt dabei vorzugsweise 0,001 bis 1000 mg, besonders bevorzugt 0,1 bis 10 mg Polymer pro Quadratmeter Oberfläche des mit dem Biozid auszurüstenden Substrats.

Das Biozid eignet sich für Substrate aus natürlichen oder künstlichen Polymeren, Papier oder Metall.

Das Polyethylenimin und Polyvinylamin eignet sich als alleiniges Biozid, d.h. der Zusatz weiterer biozider Verbindungen, z. B. von quaternären Ammoniumsalzen, wie in der WO 2004/087226 beschrieben, ist nicht notwendig.

Bei den mit dem Biozid ausgerüsteten Substraten kann es sich z.B. um Substrate für medizinische Anwendungen, Anwendungen im Sanitär- oder Hygienebereich, im Lebensmittelbereich, insbesondere bei Lebensmittelverpackungen, oder Substrate für vielfältige industrielle Anwendungen, insbesondere Filter, z.B. für Klimaanlagen, handeln.

Die Biozide können auch Wasch- und Reinigungsmitteln zugesetzt werden.

Die Biozide haben hervorragende Wirkung gegen Mikroorganismen, wie Viren, Hefen, Pilze und insbesondere gegen Bakterien.

### Beispiele

Herstellung von mit dem Biozid ausgerüsteten Papier

Aus einer 1 Gew.-% Suspension von Birke und Pinienfasern (30/70 %) wurde mit einem Rapid Köthen Papierformer ein Rohpapier mit einem Gewicht von 120 g/Quadratmeter hergestellt. Das Papier wurde auf einem zylindertrockner bei 90 °C zu einem Wassergehalt von 5 Gew.-% getrocknet und in 4 x 4 cm breite Streifen geschnitten.

Diese Streifen wurden 10 Minuten in 25 ml in eine Polyethylenimin oder Polyvinylamin-Lösung gemäß Tabelle 1 (Konzentration des Polymeren 10mg/ml) gegeben. Danach wurde die Streifen 5 Minuten in entionisiertes Wasser gegeben und dann bei 90 °C zu einem Restwassergehalt von 5 Gew.-% getrocknet.

**Tabelle 1:**

| Beispiel | Polymer | Molgewicht (Kilodalton | Ladungsdichte Meq/g bei pH 7 |
|---|---|---|---|
| Vergleich | - | - | - |
| 1 | PEI | 20000 | 8 |
| 2 | PEI | 200 | 8 |
| 3 | PEI | 20 | 8 |
| 4 | PVAm | 2000 | 18 |
| 5 | PVAm | 200 | 18 |
| 6 | PVAm | 20 | 18 |

| | | | |
|---|---|---|---|
| Abkürzungen. PEI : Polyethylenimin; PVAm: Polyvinylamin | | | |

### Biozide Wirkung

Gramnegative Bakterien (Escherichia Coli, E.C.) bzw. grampositive Bakterien (Staphylococcus Aureus, S.A.) wurden in einer Salzlösung (0,9 Gew.-% NaCl) suspendiert. Die optische Dichte der Lösung wurde bei 420 nm gemessen. Der Gehalt an E.C. bzw. S.A. betrug 1,9 mal 10 hoch 9 bzw. 2,1 mal 10 hoch 9 jeweils pro ml.

Die Papiere gemäß Tabelle 1 wurden mit 60 Minuten mit 30 ml der Bakterienlösungen inkubiert. Die Papiere wurden dann aus der Bakterien-Suspension entfernt und die optische Dichte erneut bestimmt.

**Escherichia Coli**

| Beispiel | Optische Dichte zu Anfang | Optische Dichte nach 30 Minuten | Differenz |
|---|---|---|---|
| Vergleich | 0,176 | 0,177 | 0,001 |
| 1 | 0,182 | 0,022 | 0,160 |
| 2 | 0,176 | 0,012 | 0,154 |
| 3 | 0,167 | 0,031 | 0,136 |
| 4 | 0,191 | 0,011 | 0,180 |
| 5 | 0,177 | 0,035 | 0,142 |
| 6 | 0,195 | 0,056 | 0,139 |

**Staphylococcus aureus**

| Beispiel | Optische Dichte zu Anfang | Optische Dichte nach 30 Minuten | Differenz |
|---|---|---|---|
| Vergleich | 0,176 | 0,176 | 0,001 |
| 1 | 0,173 | 0,033 | 0,14 |
| 2 | 0,180 | 0,044 | 0,136 |
| 3 | 0,170 | 0,021 | 0,149 |
| 4 | 0,182 | 0,052 | 0,130 |
| 5 | 0,176 | 0,012 | 0,164 |
| 6 | 0,193 | 0,041 | 0,152 |

Die Abnahme der optischen Dichte ist ein Zeichen für das Absterben der Bakterien und die damit verbundene Auflösung der Bakterien.

## Patentansprüche

1. Verwendung eines kationischen Polymeren als biozider Wirkstoff, **dadurch gekennzeichnet, dass** keine sonstigen bioziden Wirkstoffe, insbesondere keine quarternären Ammoniumsalze, mitverwendet werden, es sich bei dem Polymer um ein Polyethylenimin handelt, welches zu mindestens 95 Gew.-% aus Einheiten der Formel
-CH2-CH2-N I
besteht, worin N für ein primäres, sekundäres, tertiäres oder quarternäres Stickstoffatom steht, oder um ein ein Polyvinylamin handelt, welches aus mindestens 95 Gew.-% ethylenisch ungesättigten, radikalisch polymerisierbaren Verbindungen mit einer primären, sekundären, tertiären oder quarternären Aminogruppe in polymerisierter Form besteht und das Polymer 0,1 bis 22 Milliequivalent kationische Gruppen pro Gramm Polymer enthält.

2. Verwendung gemäß Anspruch 1, **dadurch gekennzeichnet, dass** das Polymer in Form einer wässrigen Lösung oder Dispersion verwendet wird.

3. Biozid ausgerüstete Substrate **dadurch** erhältlich, dass die gewünschten Substrate mit dem Polethylenimin oder dem Polyvinylamin gemäß Anspruch 1, bzw. dessen Lösung oder Dispersion, beschichtet, imprägniert oder sonst wie behandelt werden, wobei keine sonstigen bioziden Wirkstoffe mitverwendet werden und die Substrate 0,001 bis 1000 mg Polymer pro Quadramater Oberfläche aufweisen.

4. Biozid ausgerüstete Substrate gemäß Anspruch 3, **dadurch gekennzeichnet, dass** es sich um Substrate aus natürlichen oder künstlichen Polymeren, Papier oder Metall handelt.

## Claims

1. The use of a cationic polymer as a biocidal active substance, wherein no other biocidal active substances, in particular no quaternary ammonium salts, are concomitantly used, the polymer is a polyethylenimine which comprises at least 95% by weight of units of the formula
-CH2-CH2-N I
in which N is a primary, secondary, tertiary or quaternary nitrogen atom, or a polyvinylamine which comprises at least 95% by weight of ethylenically unsaturated compounds capable of free radical polymerisation and having a primary, secondary, tertiary or quaternary amino group in polymerized form, and the polymer comprises from 0.1 to 22 milliequivalents of cationic groups per gram of polymer.

2. The use according to claim 1, wherein the polymer is used in the form of an aqueous solution or dispersion.

3. A biocidally treated substrate obtainable by coating, impregnating or otherwise treating the desired substrate with the polyethylenimine or the polyvinylamine according to claim 1 or the solution or dispersion thereof, no other biocidal active substances being concomitantly used and the substrate having from 0.001 to 1000 mg of polymer per square meter of surface.

4. The biocidally treated substrate according to claim 3, which is a substrate comprising natural or synthetic polymers, paper or metal.

## Revendications

1. Utilisation d'un polymère cationique en tant que substance active biocide, **caractérisée en ce qu'**en même temps on n'utilise pas d'autres substances actives biocides, en particulier aucun sel d'ammonium quaternaire, en ce qui concerne le polymère il s'agit d'une polyéthylène-imine qui est constituée à raison d'au moins 95 % en poids de motifs de formule
-CH₂-CH₂-N I
dans laquelle N représente un atome d'azote primaire, secondaire, tertiaire ou quaternaire, ou d'une polyvinylamine qui est constituée d'au moins 95 % en poids de composés polymérisables par voie radicalaire, à insaturation éthylénique, comportant un groupe amino primaire, secondaire, tertiaire ou quaternaire sous forme polymérisée et le polymère contient de 0,1 à 22 milliéquivalents de groupes cationiques par gramme de polymère.

2. Utilisation selon la revendication 1, **caractérisée en ce que** le polymère est utilisé sous forme d'une solution ou dispersion aqueuse.

3. Substrats traités par biocide, pouvant être obtenus par enduction, imprégnation ou traitement d'une autre façon des substrats désirés par la polyéthylène-imine ou la polyamine selon la revendication 1 ou sa solution ou dispersion, aucune autre substance active biocide n'étant en même temps utilisée et les substrats comportant de 0,001 à 1 000 mg de polymère par mètre carré de surface.

4. Substrats traités par biocide selon la revendication 3, **caractérisés en ce qu'**il s'agit de substrats à base de polymères naturels ou synthétiques, de papier ou de métal.
